# EUROPEAN PATENT SPECIFICATION

(11) **EP 0 846 094 B1**
(45) Date of publication and mention of the grant of the patent: **25.04.2001**
(21) Application number: 96921717.3
(22) Date of filing: 20.06.1996
(51) Int. Cl.: C07C 43/11, C07C 305/06, C11D 1/29

(54) **BRANCHED CHAIN COMPOUNDS AND THEIR USE**
VERZWEIGTKETTIGE VERBINDUNGEN UND IHRE VERWENDUNG
COMPOSES A CHAINE RAMIFIEE ET LEUR UTILISATION

(30) Priority: 20.06.1995 US 493189
(43) Date of publication of application: 10.06.1998
(73) Proprietor: ALBEMARLE CORPORATION, Baton Rouge, LA 70801-1765 (US)
(72) Inventor: SAUER, Joe, D., Baton Rouge, LA 70816 (US); ZAWESKI, Edward, F., Aurora, IL 60504 (US); TUVELL, Melvin, E., Baton Rouge, LA 70810 (US); TROWBRIDGE, Francis, A., Liberty, MO 64068 (US); BUNCH, David, W., St. Francisville, LA 70075 (US)
(74) Representative: UEXKÜLL & STOLBERG
(86) International application number: US9610634
(87) International publication number: WO9700843

(56) References cited:
- WO-A-92/20773
- US-A- 3 887 624
- JOURNAL OF COLLOID AND INTERFACE SCIENCE, vol. 147, no. 2, December 1991, pages 403-406, XP000602370 R. VARADARAJ ET AL: "Relationships between dynamic contact angle and dynamic surface tension properties for linear and branched ethoxylate, ethoxysulfate, and sulfate surfactants"

## Description

This invention relates to certain branched chain compounds having surfactant properties enabling their use as wetting agents, solubilizing agents, washing agents, dispersing agents, emulsifying agents, and/or scouring agents, and novel intermediates therefor.

### Background

In U.S. Pat. Nos. 3,887,624 and 3,952,068, R. M. Gipson, F. E. Bentley and J. G. Milligan describe vinylidene alcohol compositions having improved properties for surfactant uses as compared to similar prior linear alcohol-based surfactants and prior highly branched alcohol-based surfactants. In addition to primary vinylidene alcohols, these patents disclose ethoxylates of vinylidene alcohols having the formula R_{z}''''-(OC₂H₄)_{w}-OH where z is the number of carbon atoms in the alkyl group, R'''', and w is 7 to 15 and preferably 7 to 12, and represents the average number of oxyethylene groups present in the ethoxylate. Stressed in the patents is the desirability of having the ratio of w to z within the range of 0.685 to 0.755, and also of having z equal to 13 and w in the range of 9 to 10. These two patent disclosures are fully incorporated herein by reference for all purposes.

### The Invention

This invention provides as a first embodiment, useful nonionic ethoxylates of certain vinylidene alcohols having 1 to 6.5 oxyethylene groups per molecule, and a ratio of the number of ethyleneoxy groups (w) to carbon atoms in the alkyl group (z) of 0.433 or less.

These compounds of the invention have the formula:

(I) R_{z}-(OC₂H₄)_{w}-OH

where R is an alkyl group which is bifurcated at the 3-position and each branch has at least 4 carbon atoms (preferably at least 5 carbon atoms, and most preferably at least 6 carbon atoms); z is the number of carbon atoms in R, and is 15 to 33 (preferably 17 to 25); and w is 1 to 6.5 (preferably 2 to 6). It will be seen that the highest ratio of w to z exists when w is 6.5 and z is 15, whereby the ratio w/z is 0.433. Depending on the preparative and if any, recovery and/or purification procedures employed in forming compounds of formula (I), they may be associated with minor amounts (i.e., less than 50 mol%) of other isomers or byproducts. As long as the produced, recovered or purified product contains more than 50 mol% of compound(s) of the above formula, it is are suitable in the practice of this invention. Preferred products as produced, recovered or purified contain at least 70 mol% of compound(s) of formula (I), and particularly preferred products as produced, recovered or purified contain at least 90 mol% of compound(s) of formula (I). In all cases, however, the requirements remain that w for the product as produced, recovered or purified is 6.5 or less, and that the w/z ratio of such product as produced, recovered or purified does not exceed 0.433.

Formula (II) further illustrates the structure of the major isomer (50 mol% or more) of the compounds of this first embodiment of the invention: 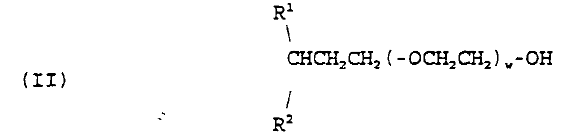 where R¹ and R² are straight chain alkyl groups each having at least 4 carbon atoms (preferably at least 5 carbon atoms, and most preferably at least 6 carbon atoms) and the total number of carbon atoms in R¹ and R² is from 12 to 30 (preferably 14 to 22), and w is as defined above.

To prepare the products of this invention, the following method can be used. It starts with an olefin and comprises:
a) dimerization of a straight chain 1-alkene using an aluminum alkyl dimerization catalyst to form a vinylidene olefin having one carbon atom less than the desired value for z in formulas (I) and (III) above;
b) hydroformylation of the vinylidene olefin under typical hydroformylation (oxo) reaction conditions involving reaction with carbon monoxide and hydrogen, and a suitable catalyst (which typically is a complex of cobalt carbonyl or rhodium carbonyl) to produce a branched chain primary alcohol having one additional carbon atom in the 1-position; and
c) ethoxylation of the branched chain primary alcohol with ethylene oxide under typical ethoxylation reaction conditions to form a product of formula (I).

Other embodiments and features of this invention appear hereinafter.

The compounds of this invention have a "double tailed" configuration in a hydrophobic alkyl group, and hydrophilic functionality at the end of the molecule. Hydrophobic and hydrophilic balance is a property of importance for surfactant utilities. Instead, these double tailed surfactants tend to distribute as bilayers and afford liquid crystal systems. At a broad range of conditions, these liquid crystal systems are capable of providing very low interfacial tensions which directly results in their desirable performance features.

A method which can be used to synthesize the compounds of this invention is illustrated by the following examples. A two-step hydroformylation reaction for converting a vinylidene olefin into a primary oxo alcohol is illustrated in Example 1.

### Example 1

### Synthesis of Intermediate

A 1-liter autoclave is charged with 336 g (1.5 mol) of distilled 2-hexyl-1-decene formed by dimerization of 1-octene using tri-n-octylaluminum catalyst, 10.1 g (0.0295 mol) of Co₂(CO)₈ and 131 g of toluene. The autoclave is sealed, flushed with synthesis gas (H₂/CO) , and pressured to 13969 kPa gauge (2026 psig) at 32°C with synthesis gas and heated to 130°C. Autoclave temperature is maintained at 130°C while synthesis gas pressure is maintained at 20684 kPa (gauge) (3000 psig) during repressurization. After 21 hours the autoclave contents are cooled to 32°C, vented and flushed with hydrogen. The autoclave is then pressured with hydrogen 16341 kPa (gauge) (2370 psig) and heated to 190°C. The pressure is then increased to 21663 kPa (gauge) (3142 psig) with hydrogen and held under these conditions for 18 hours. The autoclave contents are then cooled to room temperature, vented, flushed with nitrogen, and discharged. The product mixture is subjected to fractional distillation to recover a fraction boiling in the range of 97-110°C at 0.0133 - 0.0533 kPa (0.1-0.4 mm of Hg pressure). In a reaction conducted in accordance with the forgoing procedure a product, shown by nmr to contain 96.3 mol % of primary alcohols, was recovered. The major portion of the product corresponds to 3-hexyl-1-undecanol.

Example 2 illustrates a one-step hydroformylation procedure for producing a primary oxo alcohol for use in preparing a product of this invention.

### Example 2

### Synthesis of Intermediate

To a 1-liter autoclave are charged 336 g (1.5 mol) of distilled 2-hexyl-1-decene formed by dimerization of 1-octene using tri-n-octylaluminum catalyst, 10.1 g (0.0295 mol) of Co₂(CO)₈, 13.03 g (0.0590 mol) of 9-phenyl-9-phosphabicyclononane, and 146 g of toluene. The autoclave is sealed, flushed with a mixture of hydrogen and carbon monoxide containing approximately 14 wt% hydrogen (2H₂/CO), pressured to 7564 kPa (gauge) (1097 psig) at 22°C, and heated to and held at 200°C. Pressure uptake occurs at about 190°C. When the pressure drops to 10343 kPa (gauge) (1500 psig), the autoclave is repressured to 13700 kPa (gauge) (2000 psig) with the same synthesis gas mixture. After 21 hours the autoclave contents are cooled to room temperature, vented and flushed with nitrogen. The autoclave is then pressured with hydrogen to 16341 kPa (gauge) (2370 psig) and heated to 190°C. The pressure is then increased to 21663 kPa (gauge) (3142 psig) with hydrogen and held under these conditions for 18 hours. The autoclave contents are then cooled to room temperature, vented, flushed with hydrogen, and discharged. The product mixture is stripped of toluene and subjected to fractional distillation to recover a fraction boiling in the range of 120-126°C at 0.02-0.007 kPa 0.15-0.05 mm of Hg pressure). In a reaction conducted in accordance with the forgoing procedure, a product shown by vpc to contain 96.2 area % of primary alcohols was formed. NMR indicated that 75 mol % of the product was 3-hexyl-1-undecanol, along with 22.8 mol % of an isomeric branched chain primary alcohol. The balance (2.2 mol %) was formate ester.

Example 3 illustrates a typical ethoxylation procedure for producing a product of formula (II) of this invention by ethoxylation.

### Example 3

### Mixed Ethoxylates of 3-Hexyl-1-Undecanol (5:1 Mol Ratio)

To an autoclave are charged 0.5 mol of 3-hexyl-1-undecanol as the 75 mol % product formed as in Example 2, and 0.5 g of potassium hydroxide flakes. While holding the temperature at 140°C, ethylene oxide is slowly charged until 110 g (2.5 mols) of ethylene oxide has been charged. The KOH catalyst is neutralized with oxalic acid, and the product mixture is filtered using filter aid.

Examples 4-11 illustrate the Williamson ether synthesis procedure which can be used.

### Example 4

### Synthesis of Intermediate

To a 500 mL round bottom flask containing 102.4 (0.4 mol) of C₁₇H₃₅OH (oxo alcohol) formed as in Example 2 and 1.0 g (0.0064 mol) of C₁₀H₂₁NH₂ (decylamine) is added 57.1 g (0.48 mol) of SOCl₂ (thionyl chloride) at 80°C over a three (3) hour period. Reaction is allowed to continue overnight at 80°C. The reaction mixture is taken up in benzene and washed with water. The organic layer is separated and rotary evaporated at aspirator pressure and 90°C. The organic reaction residue is fractionally distilled to give product boiling at 117-123°C at 0.05 mm Hg pressure. The product is indicated by GC/mass spectral analysis to contain two isomeric forms of C₁₇H₃₅Cl. The main isomer is C₈H₁₇(C₆H₁₃)CH-CH₂-CH₂Cl. The other isomer is C₇H₁₅CH(CH₂Cl) -CH-C₆H₁₃-CH₃.

### Example 5

### Triethoxylate of 3-Hexyl-1-Undecanol

To a reaction flask under a nitrogen atmosphere containing 52.86 g (0.35 mols) of dried H(OCH₂CH₂)₃OH (triethylene glycol) is slowly added pieces of potassium metal (3.42 g, 0.088 mol). After potassium addition is completed, the reaction mass is heated at 100°C for 4 hours. Reaction mass temperature is then increased to 140°C and 20.06 g (0.073 mol) of C₁₇H₃₅Cl from Example 4 is added over 3 hours. Reaction is allowed to continue overnight. GC shows most of the chloride has reacted to give a small amount of C₁₇ olefin but mostly the desired C₁₇H₃₅(OCH₂CH₂)₃OH where the C₁₇ alkyl group of the principal isomer is C₈H₁₇(C₅H₁₃)CH-CH₂-CH₂-. The reaction mass is diluted with benzene and washed with water to remove residual triethylene glycol. The benzene solution is rotary evaporated under aspirator pressure and 90°C to remove the benzene. The organic residue is fractionally distilled to give the following a purer product of Formula (II) above, boiling in the range of 187-197°C, mainly C₈H₁₇(C₆H₁₃)CHCH₂CH₂-(OCH₂CH₂)₃OH.

### Example 6

### Pentaethoxylate of 3-Hexyl-1-Undecanol

To a 500 mL round bottom flask equipped with stirrer, condenser, thermometer and addition funnel is added 221.56 g (0.93 mol) of pentaethylene glycol (H(OCH₂CH₂)₅OH. To this is added 9.36 g (0.24 mol) of potassium metal pieces over 1/2 hour and then the system is heated to 80°C. The reaction mass is stirred at 80°C until the reaction mass is amber and homogeneous. To this reaction mass at 140°C is added over a period of 60 minutes, 54.9 g (0.2 mol) of C₁₇H₃₅Cl (oxo chloride) formed as in Example 4. The reaction temperature is increased to 160°C for 16 hours, then increased to 180°C for 8 hours, and then again increased and held at 200°C for 16 hours. The reaction mass is cooled to room temperature, diluted with benzene and washed with water to remove residual pentaethylene glycol. The benzene solution is rotary evaporated to remove the benzene, and then is fractionally distilled. The desired product is recovered in the ranges of from 224°C at 0.0067 kPa (0.05 mm Hg) to 248°C at 0.0267 kPa (0.2 mm Hg). The product is composed of two isomers, one of which is C₈H₁₇(C₅H₁₃)CHCH₂CH₂-(OCH₂CH₂)₅OH.

### Example 7

### Synthesis of Intermediate

To a 3-neck round bottom flask is added 15.3 g (0.32 mol) of C₁₇H₃₅ (OCH₂CH₂)₅OH and 0.08 g (0.0005 mol) of C₁₀H₂₁NH₂. This is heated to 80°C and 4.59 g (0.0386 mol) of thionyl chloride (SOCl₂) is added over 2 hours and allowed to continue at this temperature for 5 hours. The reaction mixture is cooled to room temperature, diluted with pentane and water washed. The pentane solution is dried over anhydrous MgSO₄ and rotary evaporated to give over 90% yield of product indicated by GC/mass spectral data to be consistent with C₁₇H₃₅(OCH₂CH₂)₄-O-CH₂CH₂Cl.

### Example 8

### 6.4-Ethoxylate of 3-Hexyl-1-Undecanol

To a reaction flask containing dried diethylene glycol, H(OCH₂CH₂)₂OH, 13.59 g (0.128 mol) is added 1.25 g (0.032 mol) of potassium metal. The mixture is stirred for 3 hours at ambient temperature and then for 4 hours at 130°C. The temperature is raised to 160°C and 14.74 g (0.0298 mol) of product of Example 7, C₁₇H₃₅(OCH₂CH₂)₄-O-CH₂CH₂Cl, is added over 2 hours. The temperature is held at 160°C for 12 hours, at 180°C for 24 hours, at 200°C for 24 hours, and finally at 220°C for 48 hours. The reaction mass is cooled to room temperature, diluted with hexane, washed with water, dried over anhydrous MgSO₄, filtered and rotary evaporated at aspirator pressure and 90°C. The organic residue is Kugel-Rohr distilled up to 170°C and 0.0533 kPa 0.4 mm Hg to give a forecut and a pot residue. The pot residue is indicated by GC/mass spectral analysis to be C₁₇H₃₅(OCH₂CH₂)_{6,4}OH, which thus contains the desired isomer C₈H₁₇ (C₆H₁₃) CHCH₂CH₂(OCH₂CH₂)_{6.4}OH.

### Example 9

### Tetraethoxylate of 3-Hexyl-1-Undecanol

To a reaction flask containing distilled tetraethylene glycol, H(OCH₂CH₂)₄OH, 114 g (0.58 mol) is added 5.75 g (0.147 mol) of potassium metal over a period of 2 hours while maintaining temperature below 65°C. After potassium has reacted, the temperature is raised to 140°C while C₁₇H₃₅Cl (oxo chloride, formed as in Example 4) is added over 2 hours. The temperature is increased to 160°C and kept there for 24 hours. The reaction mass is cooled to room temperature, dissolved in benzene and washed with warm water. The benzene solution is then rotary evaporated and then fractionally distilled to give the desired product: C₁₇H₃₅(OCH₂CH₂)₄OH mainly as two isomers one of which is C₈H₁₇ (C₆H₁₃) CHCH₂CH₂(OCH₂CH₂)₄OH.

### Example 10

### Diethoxylate of 3-Hexyl-1-Undecanol

To a round bottom flask is added 42.4 (0.4 mol) of diethylene glycol under a nitrogen atmosphere. To this reaction mass is added 2.3 g (0.1 mole) of sodium over 1 hour. The reaction mass is heated to 130°C whereby all of the sodium has reacted. Then 27.4 g (0.1 mol) of C₁₇H₃₅Cl is added over 1 hour and then kept for 17 hours at these conditions. Reaction temperature is then increased to 140°C and kept for 17 hours at these conditions for 24 hours. The reaction mass upon cooling consists of 2 layers. GC shows the bottom layer to be mostly diethylene glycol. The reaction mass is filtered and the bottom diethylene glycol layer separated. The top organic layer is diluted with diethyl ether, water washed and dried over anhydrous MgSO₄. After filtering, the organic layer is fractionally distilled to give a fraction at from 200-220°C and 0.3 - 0.33 kPa (2.25 - 2.5 mm Hg) containing the desired product, C₁₇H₃₅O(CH₂CH₂O)₂OH, in two isomeric forms, one of which is the desired isomer of this invention, namely C₈H₁₇(C₆H₁₃)CHCH₂CH₂-(OCH₂CH₂)₂OH.

## Claims

1. A compound of the formula R_{z}-(OC₂H₄)_{w}-OH where R has the formula: 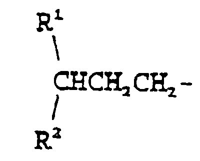 in which R¹ and R² are both straight chain alkyl branches and each such branch has at least 4 carbon atoms with the total number of carbon atoms in R¹ and R² being 12 to 30; wherein z is the total number of carbon atoms in R and is 15 to 33; wherein w is 1 to 6.5; and wherein the ratio of w/z does not exceed 0.433:1.

2. A compound of claim 1 wherein each said branch has at least 5 carbon atoms.

3. A compound of claim 1 wherein each said branch has at least 6 carbon atoms.

4. A compound of claim 1 wherein z is 17 to 25.

5. A compound of claim 1 wherein w is 2 to 6.

6. A compound of claim 1 wherein each said branch has at least 6 carbon atoms, wherein z is 17 to 25, and wherein w is 2 to 6.

7. A compound of claim 1 having the formula
C₈H₁₇ (C₆H₁₃) CHCH₂CH₂ (OCH₂CH₂)₂OH.

8. A compound of claim 1 having the formula
C₈H₁₇(C₆H₁₃)CHCH₂CH₂(OCH₂CH₂)₃OH.

9. A compound of claim 1 having the formula
C₈H₁₇ (C₆H₁₃) CHCH₂CH₂ (OCH₂CH₂)₄OH.

10. A compound of claim 1 having the formula
C₈H₁₇ (C₆H₁₃) CHCH₂CH₂ (OCH₂CH₂)₅OH.

11. A compound of claim 1 having the formula
C₈H₁₇ (C₆H₁₃)CHCH₂CH₂ (OCH₂CH₂) _{6,4}OH.

12. A process for producing a compound of the formula R_{z}-(OC₂H₄)_{w}-OH where R has the formula: 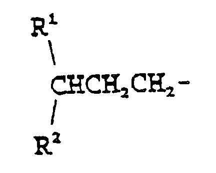 in which R¹ and R² are both straight chain alkyl branches and each such branch has at least 4 carbon atoms with the total number of carbon atoms in R¹ and R² being 12 to 30; wherein z is the total number of carbon atoms in R and is 15 to 33; wherein w is 1 to 6.5; and wherein the ratio of w/z does not exceed 0.433:1, which process comprises:
a) dimerizing a straight chain 1-alkene using an aluminum alkyl dimerization catalyst to form a vinylidene olefin having 1 carbon atom less than the desired value for z in the foregoing formula;
b) hydroformulating said vinylidene olefin with carbon monoxide and hydrogen in the presence of a hydroformulation catalyst to produce a branched chain primary alcohol having one additional carbon atom in the 1-position; and
c) ethoxylating said branched chain primary alcohol with ethylene oxide in an amount to produce a product of the foregoing formula.

13. A process according to claim 12 wherein the straight chain 1-alkene has from 8 to 12 carbon atoms in the molecule.

14. A process according to claim 12 wherein the straight chain 1-alkene is 1-octene.

15. A process according to any of claims 12 to 14 wherein said amount of ethylene oxide is sufficient to produce a compound of the foregoing formula wherein w is in the range of 2 to 6.

## Patentansprüche

1. Verbindung mit der Formel R_{z}-(OC₂H₄)_{w}-OH, in der R die Formel: 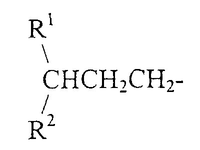 hat, in der R¹ und R² beide geradkettige Alkylzweige sind und jeder dieser Zweige mindestens 4 Kohlenstoffatome aufweist, wobei die Gesamtzahl an Kohlenstoffatomen in R¹ und R² 12 bis 30 ist, z die Gesamtzahl von Kohlenstoffatomen in R ist und 15 bis 33 ist, w 1 bis 6,5 ist und das Verhältnis von w/z 0,433:1 nicht übersteigt.

2. Verbindung nach Anspruch 1, bei der jeder der Zweige mindestens 5 Kohlenstoffatome aufweist.

3. Verbindung nach Anspruch 1, bei der jeder der Zweige mindestens 6 Kohlenstoffatome aufweist.

4. Verbindung nach Anspruch 1, bei der z 17 bis 25 ist.

5. Verbindung nach Anspruch 1, bei der w 2 bis 6 ist.

6. Verbindung nach Anspruch 1, bei der jeder der Zweige mindestens 6 Kohlenstoffatome aufweist, z 17 bis 25 ist und w 2 bis 6 ist.

7. Verbindung nach Anspruch 1 mit der Formel
C₈H₁₇ (C₆H₁₃) CHCH₂CH₂ (OCH₂CH₂)₂OH.

8. Verbindung nach Anspruch 1 mit der Formel
C₈H₁₇ (C₆H₁₃) CHCH₂CH₂ (OCH₂CH₂)₃OH.

9. Verbindung nach Anspruch 1 mit der Formel
C₈H₁₇(C₆H₁₃)CHCH₂CH₂(OCH₂CH₂)₄OH.

10. Verbindung nach Anspruch 1 mit der Formel
C₈H₁₇(C₆H₁₃)CHCH₂CH₂(OCH₂CH₂)₅OH.

11. Verbindung nach Anspruch 1 mit der Formel
C₈H₁₇(C₆H₁₃) CHCH₂CH₂ (OCH₂CH₂)_{6,4}OH.

12. Verfahren zur Herstellung einer Verbindung mit der Formel R_{z}-(OC₂H₄)_{w}-OH, in der R die Formel: 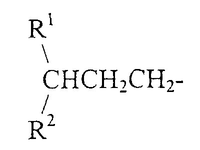 hat, in der R¹ und R² beide geradkettige Alkylzweige sind und jeder dieser Zweige mindestens 4 Kohlenstoffatome aufweist, wobei die Gesamtzahl an Kohlenstoffatomen in R¹ und R² 12 bis 30 ist, z die Gesamtzahl von Kohlenstoffatomen in R ist und 15 bis 33 ist, w 1 bis 6,5 ist und das Verhältnis von w/z 0,433:1 nicht übersteigt, wobei das Verfahren umfaßt:
a) Dimerisierung von geradkettigem 1-Alken unter Verwendung eines Aluminiumalkyldimerisationskatalysators unter Bildung von Vinylidenolefin mit einem Kohlenstoffatom weniger als der gewünschte Wert für z in der zuvor genannten Formel,
b) Hydroformulierung des Vinylidenolefins mit Kohlenmonoxid und Wasserstoff in Gegenwart eines Hydroformulierungskatalystors zur Herstellung eines verzweigten primären Alkohols mit einem zusätzlichen Kohlenstoffatom in der 1-Position, und
c) Ethoxylierung des verzweigten primären Alkohols mit Ethylenoxid in einer Menge, um ein Produkt der zuvor genannten Formel herzustellen.

13. Verfahren nach Anspruch 12, bei dem das geradkettige 1-Alken 8 bis 12 Kohlenstoffatome im Molekül aufweist.

14. Verfahren nach Anspruch 12, bei dem das geradkettige 1-Alken 1-Octen ist.

15. Verfahren nach einem der Ansprüche 12 bis 14, bei dem die Menge an Ethylenoxid ausreicht, um eine Verbindung der zuvor genannten Formel herzustellen, in der w im Bereich von 2 bis 6 liegt.

## Revendications

1. Composé de formule R_{z}-(OC₂H₄)_{w}-OH dans laquelle R a pour formule: 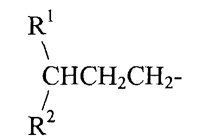 dans laquelle R¹ et R² sont tous deux des ramifications alkyle à chaîne droite et chacune de ces ramifications comporte au moins 4 atomes de carbone, le nombre total d'atomes de carbone dans R¹ et R² étant de 12 à 30; dans laquelle z est le nombre total d'atomes de carbone de R et vaut 15 à 33; dans laquelle w a une valeur de 1 à 6,5; et dans laquelle le rapport w/z ne dépasse pas 0,433:1.

2. Composé selon la revendication 1, dans lequel chacune de ces ramifications comporte au moins 5 atomes de carbone.

3. Composé selon la revendication 1, dans lequel chacune de ces ramifications comporte au moins 6 atomes de carbone.

4. Composé selon la revendication 1, dans lequel z a une valeur de 17 à 25.

5. Composé selon la revendication 1, dans lequel w a une valeur de 2 à 6.

6. Composé selon la revendication 1, dans lequel chacune desdites ramifications comporte au moins 6 atomes de carbone, dans lequel z a une valeur de 17 à 25 et dans laquelle w a une valeur de 2 à 6.

7. Composé selon la revendication 1, de formule
C₈H₁₇(C₆H₁₃)CHCH₂CH₂(OCH₂CH₂)₂OH.

8. Composé selon la revendication 1, de formule
C₈H₁₇(C₆H₁₃)CHCH₂CH₂(OCH₂CH₂)₃OH.

9. Composé selon la revendication 1, de formule
C₈H₁₇(C₆H₁₃)CHCH₂CH₂(OCH₂CH₂)₄OH.

10. Composé selon la revendication 1, de formule
C₈H₁₇(C₆H₁₃)CHCH₂CH₂(OCH₂CH₂)₅OH.

11. Composé selon la revendication 1, de formule
C₈H₁₇(C₆H₁₃)CHCH₂CH₂(OCH₂CH₂)_{6,4}OH.

12. Procédé de production d'un composé de formule R_{z}-(OC₂H₄)_{w}-OH dans laquelle R a pour formule: 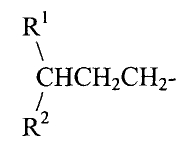 dans laquelle R¹ et R² sont tous deux des ramifications alkyle à chaîne droite et chacune de ces ramifications comporte au moins 4 atomes de carbone, le nombre total d'atomes de carbone dans R¹ et R² étant de 12 à 30; dans laquelle z est le nombre total d'atomes de carbone de R et vaut 15 à 33; dans laquelle w a une valeur de 1 à 6,5; et dans laquelle le rapport w/z ne dépasse pas 0,433:1, ce procédé comprenant:
a) la dimérisation d'un 1-alcène à chaîne droite en utilisant un catalyseur de dimérisation de type alkylaluminium pour former une oléfine vinylidène comportant un atome de carbone de moins que la valeur souhaitée pour z dans la formule ci-dessus;
b) l'hydroformylation de ladite oléfine vinylidène avec du monoxyde de carbone et de l'hydrogène, en présence d'un catalyseur d'hydroformylation, pour produire un alcool primaire à chaîne ramifiée comportant au moins un atome de carbone de plus en position 1; et
c) l'éthoxylation dudit alcool primaire à chaîne ramifiée avec de l'oxyde d'éthylène en une quantité permettant de former un produit de formule ci-dessus.

13. Procédé selon la revendication 12, dans lequel le 1-alcène à chaîne droite comporte de 8 à 12 atomes de carbone dans la molécule.

14. Procédé selon la revendication 12, dans lequel le 1-alcène à chaîne droite est le 1-octène.

15. Procédé selon l'une quelconque des revendications 12 à 14, dans lequel ladite quantité d'oxyde d'éthylène suffit pour produire un composé de formule ci-dessus, dans laquelle w est dans la gamme de 2 à 6.
